# EUROPÄISCHE PATENTSCHRIFT

(11) **EP 1 418 171 B1**
(45) Veröffentlichungstag und Bekanntmachung des Hinweises auf die Patenterteilung: **14.12.2011**
(21) Anmeldenummer: 03024921.3
(22) Anmeldetag: 29.10.2003
(51) Int. Cl.: C07C 233/15, C07C 231/12, C07C 211/52, C07C 209/74

(54) **Process for the preparation of perfluoralkylanilines**
Verfahren zur Herstellung von Perfluoroalkylanilins
Procédé pour la préparation de perfluoroalkylanilines

(30) Priorität: 11.11.2002 DE 10252275
(43) Veröffentlichungstag der Anmeldung: 12.05.2004
(73) Patentinhaber: Saltigo GmbH, 40764 Langenfeld (DE)
(72) Erfinder: Marhold, Albrecht, Dr., 51373 Leverkusen (DE); Pleschke, Axel, Dr., 51069 Köln (DE)
(74) Vertreter: Wichmann, Birgid

(56) Entgegenhaltungen:
- EP-A1- 0 936 212
- EP-A2- 1 006 102
- US-A1- 2001 041 814
- HUANG X-T ET AL: "Fluoroalkylation of aromatic compounds with per(poly)fluoroalkyl chlorides initiated by sodium dithionite in DMSO" JOURNAL OF FLUORINE CHEMISTRY, ELSEVIER SEQUOIA, LAUSANNE, CH, Bd. 111, Nr. 2, 28. Oktober 2001 (2001-10-28), Seiten 107-113, XP004308451 ISSN: 0022-1139
- WINEY D. A. ET AL.: "Elimination mechanisms. Deuteroxide/Hydroxide isotope effects as a measure of proton transfer in the transition states for E2 elimination of 2-(p-trimethylammoniophenyl)ethyl 'onium ions and halides." JOURNAL OF THE AMERICAN CHEMICAL SOCIETY., Bd. 97, Nr. 11, 1975, Seiten 3102-3108, XP002269987 DC US
- ZHOU Q. L. ET AL.: "A facile method for fluoroalkylation of aniline and its derivatives" JOURNAL OF FLUORINE CHEMISTRY., Bd. 39, Nr. 1, 1988, Seiten 87-98, XP002269988 CHELSEVIER SEQUOIA. LAUSANNE.
- YAGUPOL'SKII, L. M. ET AL: 'Perfluoroalkylation of thiazole and pyridine derivatives' JOURNAL OF GENERAL CHEMISTRY OF THE USSR , 39(9), 2041-44 ISSN: 0022-1279 1969, XP008027998

## Beschreibung

Die vorliegende Erfindung betrifft ein Verfahren zur Herstellung von Perfluoralkylanilinen, welche zur Herstellung von Wirkstoffen insbesondere für Agrochemikalien anwendbar sind.

Perfluoralkylaniline, wie insbesondere 4-Perfluoralkyl-2-methylaniline, sind wertvolle Ausgangsprodukte zur Herstellung von Insektiziden des Aroylhamstoff-Typs (siehe auch EP-A 919 542 und EP-A 936 212). Die Herstellung von Perfluoralkylanilinen kann beispielsweise durch Umsetzung von Anilinen mit Perfluoralkyliodiden oder -bromiden in aprotischen Lösungsmitteln und entweder in Gegenwart von Metallen und Schwefeldioxid (EP-A 206 951 und FR-A 2 660 923) oder in Gegenwart von Alkalimetalldithionit erfolgen (EP-A 298 803). Analog dazu können auch Perfluoralkylchloride in Dimethylsulfoxid umgesetzt werden (Huang et al., J. Fluorine Chem., 111, 2001, 107-113). Nachteilig an den genannten Methoden ist, dass die Perfluoralkyliodide oder -bromide sehr teuer sind und sich lediglich sehr geringe oder nur mäßige Ausbeuten erzielen lassen.

In einem Verfahren gemäß EP-A 1 006 102 können Perfluoralkylaniline durch Umsetzung von Anilinen mit Perfluoralkyliodiden in einem Zweiphasensystem in Gegenwart eines Reduktionsmittels erhalten werden. Allerdings ist auch hier der hohe Preis der Perfluoralkyliodide nachteilig.

Es bestand daher auch das Bedürfnis, ein Verfahren bereitzustellen, das die Herstellung von Perfluoralkylanilinen in guten Ausbeuten und in einfacher Weise ermöglicht.

Es wurde nun ein Verfahren zur Herstellung von Verbindungen der Formel (I) gefunden, 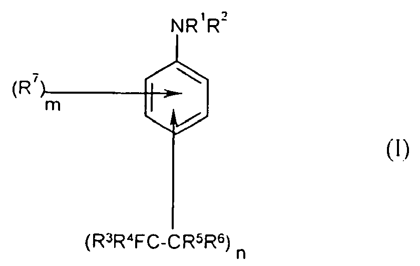 in der
- R¹ und R²: jeweils unabhängig voneinander für Wasserstoff, C₁-C₁₂-Alkyl, CO(C₁-C₁₂-Alkyl), CO(C₅-C₁₄-Aryl), CO(C₆-C₁₅-Arylalkyl), COO(C₁-C₁₂-Alkyl), COO(C₅-C₁₄-Aryl), COO(C₆-C₁₅-Arylalkyl), COO(C₂-C₁₂-Alkenyl) oder C₆-C₁₅-Arylalkyl stehen oder
- NR¹R²: als Ganzes für einen cyclischen Rest mit insgesamt 4 bis 16 Kohlenstoffatomen oder Isocyanat steht und
- R³, R⁴, R⁵ und R⁶: für Fluor oder C₁-C₁₂-Perfluoralkyl stehen und/oder jeweils zwei der Reste R³, R⁴, R⁵ und R⁶ einen cyclischen Perfluoralkylrest mit insgesamt 4 bis 20 Kohlenstoffatomen bilden und
- n: für eins oder zwei steht und
- R⁷: für C₁-C₁₂-Alkyl, C₅-C₁₄-Aryl, C₆-C₁₅-Arylalkyl, Hydroxy, Chlor, Brom, Fluor, Nitro, Cyano, freies oder geschütztes Formyl, C₁-C₁₂-Halogenalkyl oder Reste der Formeln (IIa) bis (IIf) steht,

A-B-D-E (IIa)

A-E (IIb)

A-SO₂-E (IIc)

A-B-SO₂R⁹ (IId)

A-SO₃W (IIe)

A-COW (IIf)

in denen unabhängig voneinander
A fehlt oder für einen C₁-C₈-Alkylenrest steht und
B fehlt oder für Sauerstoff, Schwefel oder NR⁸ steht, wobei
R⁸ Wasserstoff, C₁-C₈-Alkyl, C₆-C₁₅-Arylalkyl oder C₅-C₁₄-Aryl bedeutet und
D für eine Carbonyl-Gruppe steht und
E für R⁹, OR⁹, NHR¹⁰oder N(R¹⁰)₂ steht, wobei
R⁹ für C₁-C₈-Alkyl, C₁-C₈-Halogenalkyl C₆-C₁₅-Arylalkyl oder C₅-C₁₄-Aryl und
R¹⁰ jeweils unabhängig für C₁-C₈-Alkyl, C₆-C₁₅-Arylalkyl oder C₆-C₁₄-Aryl steht oder N(R¹⁰)₂ zusammen für einen cyclischen Aminorest mit 4 bis 12 Kohlenstoffatomen steht und
W für OH, NH₂, oder OM steht, wobei M ein Alkalimetallion, ein halbes Äquivalent eines Erdalkalimetallions, ein Ammoniumion oder ein organisches Ammoniumion bedeuten kann oder zwei Reste R⁷ zusammen einen cyclischen Rest mit insgesamt 5 bis 12 Kohlenstoffatomen bilden können und
m für eine ganze Zahl von 0 bis 5-n steht,
das dadurch gekennzeichnet ist, dass
- Verbindungen der Formel (II) 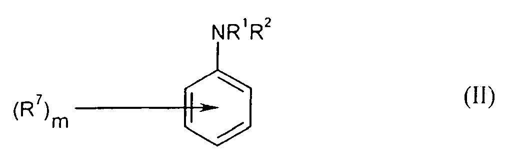 in der
   - R¹, R², R⁷ und m: die vorstehend genannte Bedeutung besitzen
- in einem Schritt a)
   mit Verbindungen der Formel (III)

   R³R⁴Hal¹C-CHal²R⁵R⁶ (III)

   in der
   - R³, R⁴, R⁵ und R⁶: die vorstehend genannte Bedeutung besitzen und
   - Hal¹ und Hal²: jeweils unabhängig voneinander für Chlor, Brom oder Iod, bevorzugt identisch für Chlor oder Brom und besonders bevorzugt identisch für Brom stehen,
   zu Verbindungen der Formel (IV) umgesetzt werden, 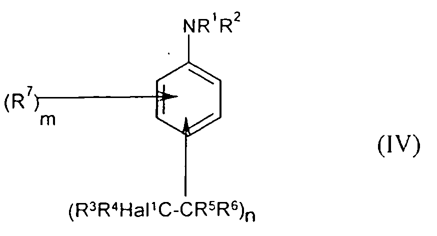 in der
   - R¹, R², R³, R⁴, R⁵, R⁶ und R⁷: sowie m, n und Hal¹ die vorstehend genannte Bedeutung besitzen und
- in einem Schritt b)
die Verbindungen der Formel (IV) durch Umsetzung mit ionischem Fluorid in Verbindungen der Formel (I) überführt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise Alkylen beziehungsweise Alkoxy beziehungsweise Alkenyl bedeutet jeweils unabhängig einen geradkettigen, cyclischen, verzweigten oder unverzweigten Alkyl- beziehungsweise Alkylen- beziehungsweise Alkoxy- beziehungsweise Alkenyl-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cycloHexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy.

C₂-C₁₂-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 1-Octenyl oder 2-Octenyl.

**Perfluoralkyl** steht jeweils unabhängig einen geradkettigen, cyclischen, verzweigten oder unverzweigten Alkylrest, der vollständig durch Fluoratome substituiert ist.

Beispielsweise und bevorzugt steht C₁-C₄-Perfluoralkyl für Trifluormethyl, Pentafluorethyl, Heptafluorisopropyl und n-Nonafluorbutyl, C₁-C₁₂-Perfluoralkyl darüber hinaus beispielsweise für Perfluorcyclopentyl, Perfluorcyclohexyl und Perfluordodecyl.

**Aryl** bedeutet jeweils unabhängig einen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 18 Gerüstkohlenstoffatomen.

Beispiele für carbocyclische aromatische Reste mit 6 bis 18 Gerüstkohlenstoffatomen sind Phenyl, Naphtyl, Phenanthrenyl, Anthracenyl oder Fluorenyl, heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyridinyl, Oxazolyl, Benzofuranyl, Dibenzofuranyl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Perfluoralkyl, COO(C₁-C₈-Alkyl), CON(C₁-C₈-Alkyl)₂, COO(C₁-C₈-Arylalkyl), COO(C₄-C₁₄-Aryl), CO(C₁-C₈-Alkyl), C₅-C₁₅-Arylalkyl oder Tri(C₁-C₆-alkyl)siloxyl.

**Arylalkyl** bedeutet jeweils unabhängig einen geradkettigen, cyclischen, verzweigten oder unverzweigten Alkyl-Rest, der einfach, mehrfach oder vollständig durch ArylReste gemäß obiger Definition substituiert sein kann.

C₆-C₁₅-Arylalkyl steht beispielsweise und bevorzugt für Benzyl.

Im Folgenden werden die bevorzugten Substitutionsmuster für Verbindungen der Formel (I) definiert:
- R¹ und R²: stehen bevorzugt jeweils identisch für Wasserstoff oder
- NR¹R²: als Ganzes für NHCO(C₁-C₁₂-Alkyl), NHCO(C₅-C₁₄-Aryl) oder NHCO(C₆-C₁₅-Arylalkyl) oder Isocyanat.
- NR¹R²: steht besonders bevorzugt als Ganzes für NH₂ oder NHCOCH₃.
- R³R⁴FC-CR⁵R⁶: als Ganzes bevorzugt für einen sekundären C₃-C₁₂-Perfluoralkylrest, besonders bevorzugt als Ganzes für Heptafluor-2-propyl, Perfluorcyclobutyl, Perfluorcyclopentyl oder Perfluorcyclohexyl.

Ganz besonders bevorzugt steht R³R⁴FC-CR⁵R⁶ als Ganzes für Heptafluor-2-propyl.
- n: steht bevorzugt für 1.
- R⁷: steht bevorzugt jeweils unabhängig für C₁-C₄-Alkyl, Chlor, Fluor, Nitro, Cyano oder C₁-C₄-Alkoxy, besonders bevorzugt für Methyl, Ethyl, Methoxy oder Ethoxy, ganz besonders bevorzugt für Methyl.
- m: steht bevorzugt für 0, 1 oder 2, besonders bevorzugt für 0 oder 1.

Eine besonders bevorzugte Verbindung der Formel (I) ist 2-Methyl-4-(heptafluor-2-propyl)-anilin.

Die als Ausgangsprodukte verwendeten Verbindungen der Formel (III) sind literaturbekannt oder analog zur Literatur synthetisierbar. In einer besonders bevorzugten Ausführungsform werden die Verbindungen der Formel (III) dadurch hergestellt, dass
Verbindungen der Formel (V)

R³-CR⁴=CR⁵-R⁶ (V)

mit Halogenverbindungen der Formel (VI)

Hal¹-Hal² (VI)

umgesetzt werden.

Dabei besitzen in den Formeln (V) und (VI) R³ , R⁴, R⁵ und R⁶ sowie Hal¹ und Hal² die unter den Formeln (I) und (III) vorstehend genannten Bedeutungen und Vorzugsbereiche.

Als besonders bevorzugte Verbindungen der Formel (III) seien genannt:
1,2-Dibrom-1,1,2,3,3,3-hexafluorpropan, 2,3-Dibrom-1,1,1,2,3,4,4,4-octafluorbutan, 1,2-Dichlor-3,3,4,4-tetrafluorcyclobutan und 1,2-Dibrom-1,2,3,3,4,4,5,5-octafluorcyclopentan, wobei 1,2-Dibrom-1,1,2,3,3,3-hexafluorpropan ganz besonders bevorzugt ist.

Gemäß Schritt a) werden Verbindungen der Formel (II) mit Verbindungen der Formel (III) zu Verbindungen der Formel (IV) umgesetzt.

Das molare Verhältnis von Verbindungen der Formel (III) zu Verbindungen der Formel (II) kann pro Äquivalent für n beispielsweise 0,7 bis 1,8 betragen, vorzugsweise 0,9 bis 1,2 und besonders bevorzugt 1,0 bis 1,1.

Die Verbindungen der Formel (IV) sind als besonders wertvolle Intermediate von der Erfindung ebenfalls umfasst. Für die Vorzugsbereiche gelten die gleichen Angaben wie für die Formeln (I) und (III).

Als Einzelverbindungen der Formel (IV) seien genannt:
4-(1-Brom-1,1,2,3,3,3-hexafluor-2-propyl)-anilin, 2-Methyl-4-(1-brom-1,1,2,3,3,3-hexafluor-2-propyl)-anilin, 2-Fluor-4-(1-brom-1,1,2,3,3,3-hexafluor-2-propyl)-anilin, 2-Chlor-4-(1-brom-1,1,2,3,3,3-hexafluor-2-propyl)-anilin, 2-Brom-4-(1-brom-1,1,2,3 ,3,3-hexafluor-2-propyl)-anilin, 2-Methyl-4-(2-bromtetrafluorethyl)-anilin, Essigsäure-2-methyl-4-(1-brom-1,1,2,3,3,3-hexafluor-2-propyl)-anilid, 2-Methyl-4-(2-brom-1,1,1,2,3,4,4,4-octafluor-3-butyl)-anilin und 2-Methyl-4-(2-brom-2,3,3,4,4,5,5-octafluorcyclo-1-pentyl)-anilin.

Die Umsetzung gemäß Schritt a) erfolgt vorzugsweise in Gegenwart eines Reduktionsmittels und/oder in Gegenwart Licht mit einer Wellenlänge von 400 nm oder weniger.

Als Reduktionsmittel sind beispielsweise geeignet: Schwefelverbindungen in den gemittelten formalen Oxidationsstufen +III, +IV und +V gegebenenfalls in Mischung mit einem Metall, das ein Standardreduktionspotential von 0 V oder weniger besitzt.

Solche Schwefelverbindungen sind beispielsweise Alkalimetalldithionite, wie Natrium- und Kaliumdithionit oder Schwefeldioxid.

Geeignete Metalle sind beispielweise Mangan, Zink oder Aluminium.

Besonders geeignete Lichtquellen, die Licht mit einer Wellenlänge von 400 nm oder weniger erzeugen, sind alle üblichen UV-Lampen, wie insbesondere Quecksilberdampf-Lampen.

Besonders bevorzugt wird Schritt a) in Gegenwart von Alkalimetalldithionit, ganz besonders bevorzugt Natriumdithionit durchgeführt.

Üblicherweise wird Schritt a) in einem flüssigen Reaktionsmedium durchgeführt.

Dazu eignen sich beispielsweise polare organische Lösungsmittel mit einem Dipolmoment [µ/D] von mindestens 2,8 und gegebenenfalls unter Zusatz von Wasser sowie mehrphasige Reaktionsmedien mit einer wässrigen und zumindest einer organischen Phase.

Besonders geeignete polare organische Lösungsmittel sind Nitrile, wie beispielsweise Acetonitril, Sulfoxide wie beispielsweise Dimethylsulfoxid, Sulfone wie beispielsweise Tetramethylensulfon und amidische Lösungsmittel wie beispielsweise Dimethylformamid, N-Methylpyrrolidon und N-Methylcaprolactam.

Besonders geeignete organische Lösungsmittel für mehrphasige Reaktionsmedien sind beispielsweise aliphatische oder aromatische, gegebenenfalls halogenierte Kohlenwasserstoffe wie beispielsweise Benzinfraktionen, Benzol, Toluol, Xylol, Chlorbenzol, Dichlorbenzol, Petrolether, Hexan, Cyclohexan, Dichlormethan, Chloroform oder Tetrachlorkohlenstoff, Ether wie beispielsweise Diethylether, Diisopropylether, tert.-Butylmethylether, Ketone wie beispielsweise Cyclohexanon, Butanon oder Methyl-isobutyl-keton und Ester wie beispielsweise Essigsäuremethylester oder Essigsäureethylester.

In einer bevorzugten Ausführungsform des erfindungsgemäßen Verfahrens wird die Reaktion in einem mehrphasigen Reaktionsmedium durchgeführt, das eine wässrige und zumindest eine organische Phase, besonders bevorzugt genau eine organische Phase aufweist.

In diesen Fällen wird die Reaktion bevorzugt in Gegenwart von Phasentransferkatalysatoren durchgeführt.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (VII) geeignet,

(Kation⁺)(Anion⁻) (VII)

in der
- (Kation⁺): für substituierte quartäre Ammonium oder Phosphonium-Kationen und
- (Anion⁻): für das Anion einer organischen oder anorganischen Säure steht.

Bevorzugte Phasentransferkatalysatoren sind solche der Formel (VII), in denen (Kation⁺) für Kationen der Formel (VIII) steht

[Pnyc(C₁-C₁₂-Alkyl)_{q}(C₆-C₁₅-Arylalkyl)ᵣ(C₅-C₁₄-Aryl)_{S}({(C₂-C₆-Alkyl)-O]_{V}-(C₁-C₆-Alkyl)}ₜ)]⁺ (VIII)

in der
- Pnyc: für Stickstoff oder Phosphor steht und
in denen jeweils (q+r+s+t) = 4 ist.

Bevorzugt steht (Anion⁻) für Fluorid, Chlorid, Bromid, Iodid, Acetat, Nitrat, Sulfat, Hydrogensulfat, Tetrafluoroborat, Hexafluorophosphat, Tosylat, und Triflat, besonders bevorzugt für Chlorid, Bromid, Iodid, Sulfat und Hydrogensulfat.

Besonders bevorzugte Phasentransferkatalysatoren sind Tetra-n-butylammoniumiodid, Tetra-n-butylammoniumbromid, Tetra-n-butylammoniumchlorid, Tri-n-butylmethylphosphoniumbromid, Tetra-n-butylammoniumhydrogensulfat, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Triethylbenzylammoniumchlorid, Methyltrioctylammoniumchlorid, Trimethylbenzylammoniumchlorid, Tetrakis-diethylaminophosphoniumchlorid, -bromid oder -iodid sowie Tris-[2-(2-methoxyethoxy)-ethyl]-amin, wobei Tetra-n-butylammoniumhydrogensulfat ganz besonders bevorzugt ist.

Die Reaktionstemperatur in Schritt a) kann beispielsweise -10°C bis zum Siedepunkt des Reaktionsmediums unter Reaktionsdruck betragen, maximal jedoch 200°C, bevorzugt ist eine Reaktionstemperatur von 0 bis 70°C.

Der Reaktionsdruck in Schritt a) kann beispielsweise 0,5 bis 100 bar betragen, Umgebungsdruck ist bevorzugt.

Vorzugsweise wird Schritt a) weiterhin in Gegenwart einer Base durchgeführt. Als Basen sind beispielsweise Erdalkali- oder Alkalimetall-hydroxide, -acetate, - phosphate, -hydrogenphosphate, -carbonate oder -hydrogencarbonate, wie beispielsweise Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, Ammoniumsalze wie beispielsweise Ammoniumacetat und Ammoniumcarbonat, Amine wie beispielsweise Trimethylamin, Triethylamin, Tributylamin, Di-isopropyl-ethylamin, Tetramethylguanidin, N,N-Dimethylanilin, Diazabicyclooctan (DABCO), Diazabicyclononen (DBN), Diazabicycloundecen (DBU), N-Methylpiperidin und Piperidin, oder aromatische Stickstoffverbindungen wie beispielsweise Pyridin, 2-, 3- und 4-N,N-Dimethylaminopyridin geeignet, wobei Alkalimetallcarbonate und -hydrogencarbonate noch weiter bevorzugt sind.

Vorzugsweise werden die Verbindungen der Formel (IV) aus dem in Schritt a) erhaltenen Reaktionsgemisch isoliert. Das kann in an sich bekannter Weise durch extraktive Verfahren mit gegebenenfalls anschließenden Reinigungsoperationen wie Destillation oder Umkristallisation erfolgen.

Sollen Verbindungen der Formel (IV) für den nachfolgenden Schritt b) eingesetzt werden, in denen die ortho-Position zur Aminogruppe durch Chlor oder Brom substituiert ist, ist es vorteilhaft, zunächst das die entsprechende in ortho-Position unsubstituierte Verbindung Schritt a) zu unterwerfen und vor Schritt b) eine Halogenierung durchzuführen.

Die Halogenierung kann dabei in an sich bekannter weise erfolgen, bevorzugt ist die Umsetzung mit N-Halogensuccinimiden in Nitrilen wie zum Beispiel Acetonitril.

In Schritt b) erfolgt die Umsetzung der Verbindungen der Formel (IV) mit ionischem Fluorid zu Verbindungen der Formel (I).

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (IX),

(Kation⁺)(F⁻) (IX)

in der (Kation⁺) die unter der Formel (VII) genannte Bedeutung einschließlich deren Vorzugsbereiche besitzt.

Gegebenenfalls können auch Mischungen von Phasentransferkatalysatoren gemäß obiger Definition und/oder Halex-Katalysatoren mit Alkalimetallfluoriden eingesetzt werden.

Halex-Katalysatoren sind beispielsweise Tetrakis(dialkylamino)phosphonium-verbindungen (WO 98/05610) oder Verbindungen der Formel (X), 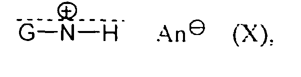 in der
- G: für einen Rest der Formeln (XIa) oder (XIb) steht 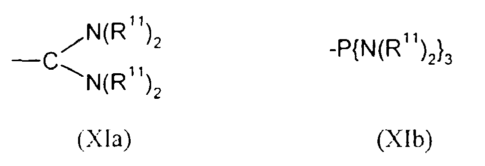 und
- H: unabhängig von G für einen Rest der Formeln (XIa), (XIb), (XIc) oder (XId)

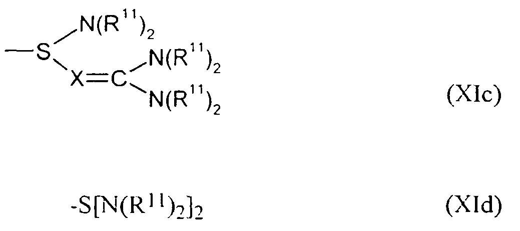

-S[N(R¹¹)₂]₂ (XId)

stehen,
wobei
die Reste R¹¹ jeweils unabhängig voneinander für C₁-C₁₂-Alkyl, C₂-C₁₀-Alkenyl oder C₆-C₁₂-Aryl stehen oder
wobei N(R¹¹)₂ als Ganzes für einen 3- bis 5-gliedrigen, gesättigten oder ungesättigten Ring stehen kann, oder
wobei die Reste der Formel (XIa) und/oder die Gruppe 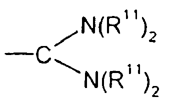 als Ganzes für einen gesättigten oder ungesättigten 4- bis 8-gliedrigen Rings stehen kann, und
- X: für Stickstoff oder Phosphor steht und
- An^{⊖}: für ein Äquivalent eines Anions wie beispielsweise und bevorzugt Chlorid, Bromid, (CH₃)₃SiF₂^{⊖}, HF₂^{⊖}, H₂F₂^{⊖}, Tetrafluoroborat, Hexafluorophosphat, Carbonat oder Sulfat steht.

Die Verbindungen der Formel (X) sind beispielsweise erhältlich durch Umsetzung von Verbindungen der Formel (XII)

[G-An'] ^{⊕}An^{⊖} (XII),

in der
- G und An^{⊖}: die bei Formel (X) angegebene Bedeutung besitzen und
- An': für Chlor oder Brom steht,
mit Verbindungen der Formel (XIII)

HN=G' (XIII),

in der
- G': hinsichtlich der Anordnung der Atome die bei Formel (X) für G angegebene Bedeutung hat, jedoch 2-bindig ist, wobei die Umsetzung in Gegenwart einer Base erfolgt.

Die Verbindungen der Formel (X) sind beschrieben in DE 101 29 057.

Für Schritt b) bevorzugt ist jedoch der Einsatz von Alkalimetallfluoriden ohne Phasentransferkatalysatoren und/oder Halex-Katalysatoren.

Bevorzugte Alkalimetallfluoride sind Natrium-, Kalium- und Cäsiumfluorid oder Mischungen davon, besonders bevorzugt ist Kaliumfluorid.

Vorzugsweise setzt man Kaliumfluorid ein, das einen Wassergehalt von 0,5 Gew.-% oder weniger, bevorzugt 0,05 Gew.-% oder weniger aufweist und eine mittlere Korngröße bezogen auf das Gewicht von 200 µm oder weniger aufweist.

Das molare Verhältnis von ionischem Fluorid zu eingesetzter Verbindung der Formel (IV) kann beispielsweise 0,7 bis 5 betragen, vorzugsweise 0,9 bis 2 und besonders bevorzugt 1,1 bis 1,7. Die Menge an ionischem Fluorid ist nach oben prinzipiell nicht begrenzt, größere Mengen sind aber unwirtschaftlich.

Vorzugsweise wird Schritt b) in Gegenwart von organischem Lösungsmittel durchgeführt. Als organische Lösungsmittel sind beispielsweise geeignet: Ketone, wie Aceton, 2-Butanon oder Methyl-isobutyl-keton; Nitrile wie beispielsweise Acetonitril, Propanitril, Benzonitril, Benzylnitril oder Butyronitril; Amide wie beispielsweise N,N-Dimethylformamid, N,N-Dimethylacetamid, N-Methyl-formanilid, N-Methyl-pyrrolidon, N-Methylcaprolactam oder Hexamethylphosphorsäuretriamid; Sulfoxide wie beispielsweise Dimethylsulfoxid, Sulfone wie beispielsweise Tetramethylensulfon, Polyether wie beispielsweise 1,4-Dioxan, Ethylenglykoldimethylether, Ethylenglykoldiethylether, Diethylenglykoldimethylether oder Diethylenglykoldiethylether oder Gemische solcher organischen Lösungsmittel.

Vorzugsweise beträgt der Wassergehalt des Lösungsmittels im Schritt b) maximal 1 Gew.-%, besonders bevorzugt maximal 0,2 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur im Schritt b) kann beispielsweise 60°C bis zum Siedepunkt des eingesetzten Lösungsmittel bei Reaktionsdruck betragen maximal jedoch 300°C, bevorzugt 110°C bis zum Siedepunkt des eingesetzten Lösungsmittel bei Reaktionsdruck maximal jedoch 200°C betragen.

Der Reaktionsdruck im Schritt b) kann beispielsweise 0,5 bis 100 bar betragen, bevorzugt sind 3 bis 25 bar.

Die Reaktionsdauer im Schritt b) kann beispielsweise 10 min bis 72 Stunden, bevorzugt 2 bis 12 Stunden betragen.

Die Aufarbeitung der Verbindungen der Formel (I) kann in an sich bekannter Weise erfolgen, üblicherweise beispielsweise durch fraktionierte Destillation direkt aus dem Reaktionsgemisch, gegebenenfalls unter vermindertem Druck.

Die erfindungsgemäß erhältlichen Verbindungen der Formel (I) eignen sich insbesondere in einem Verfahren zur Herstellung von Wirkstoffen in Agrochemikalien wie beispielsweise Insektiziden des Aroylharnstofftyps.

Ein wesentlicher Vorteil der erfindungsgemäß erhältlichen Verbindungen der Formel (I) ist, dass sie in einfacher Weise in hohen Ausbeuten aus gut verfügbaren Edukten hergestellt werden können.

### Beispiele

### Beispiel 1

### Herstellung von 1,2-Dibrom-hexafluorpropan

Es wurden bei Raumtemperatur 2357 g Brom (760 ml, 14,75 mol) vorgelegt und unter stetigem Rühren Hexafluorpropen bis zur Entfärbung eingeleitet (19 Stunden, 2400 g, 16,00 mol). Das Reaktionsgemisch wurde mit Stickstoff ausgeblasen. Auf diese Weise wurden 4710 g 1,2-Dibrom-hexafluorpropan (95 % d. Th.) erhalten.

### Beispiel 2

### Herstellung von 2-Methyl-4-(1-brom-1,1,2,3,3,3-hexafluor-2-propyl)-anilin

Zu einer Mischung aus 900 ml Wasser, 350 ml tert.-Butylmethylether, 74,48 g (0,887 mol) Natriumhydrogencarbonat und 12,68 g Tetra-n-butylammoniumhydrogensulfat wurden bei Raumtemperatur zunächst 154,36 g (0,887 mol) Natriumdithionit und anschließend 50 g (0,467 mol) o-Toluidin (2-Methylanilin) gegeben. Anschließend wurde bei 35-40°C tropfenweise mit einer Lösung von 274,684 g 1,2-Dibrom-1,1,2,3,3,3-hexafluorpropan in 100 ml tert.-Butylmethylether versetzt und nach beendeter Zugabe 18 h bei 40°C nachgerührt. Es wurde falls notwendig mit Natriumcarbonat auf einen pH-Wert von 5 eingestellt und die organische Phase abgetrennt, getrocknet und eingeengt. Der verbleibende Rückstand wird im Vakuum bei 0.3 mbar bis 100°C Innentemperatur andestilliert

Auf diese Weise wurden 82 g (53 % d. Th.) des Produktes erhalten.

### Beispiel 3

### Herstellung von 2-Methyl-4-(heptafluor-2-propyl)-acetanilid

10 g (26,5 mmol) 2-Methyl-4-(1-brom-1,1,2,3,3,3-hexafluor-2-propyl)-acetanilid und 3 g (53 mmol) geglühtes Kaliumfluorid werden in 100 ml NMP gegeben. Die Suspension wird in einem Druckbehälter bei 3 bar Stickstoff unter Rühren 4 h auf 175°C erwärmt. NMP wird im Vakuum soweit als möglich abdestilliert, der Rückstand auf 50 ml Wasser gegeben und mit Dichlormethan dreimal extrahiert. Man erhält eine Produktmischung aus 2-Methyl-4-(heptafluor-2-propyl)-acetanilid und 2-Methyl-4-(1-brom-1,1,2,3,3,3-hexafluor-2-propyl)-acetanilid.

### Beispiel 4

### Herstellung von 2-Methyl-4-(2-bromtetrafluorethyl)-anilin

Zu einer Mischung aus 700 ml Wasser, 350 ml tert.-Butylmethylether, 78,4 g (0,93 mol) Natriumhydrogencarbonat und 38 g Tetra-n-butylammoniumhydrogensulfat wurden bei Raumtemperatur zunächst 162,5 g (0,93 mol) Natriumdithionit und anschließend 50 g (0,47 mol) o-Toluidin (2-Methylanilin) und 242,47 g (0,47 mol) 1,2-Dibrom-tetrafluorethan gegeben. Es wurde bei Raumtemperatur 24 h nachgerührt. Es wurde falls notwendig mit Natriumcarbonat auf einen pH-Wert von ca. 5 eingestellt und die organische Phase abgetrennt, getrocknet und eingeengt.

Auf diese Weise wurden 104 g (78 % d. Th.) eines Produktgemisches mit der Zusammensetzung 71 % 4-(2-Bromtetrafluorethyl)-2-methylanilin, 13 % 2-(2-Bromtetrafluorethyl)-6-methylanilin und 16 % 2,4-Di-(2-bromtetrafluorethyl)-6-methylanilin erhalten.

### Beispiel 5

### Herstellung von 4-(1-Brom-1,1,2,3,3,3-hexafluor-2-propyl)-anilin

Zu einer Mischung aus 4000 ml Wasser, 1200 ml tert.-Butylmethylether, 270,61 g (3,22 mol) Natriumhydrogencarbonat und 10,94 g Tetra-n-butylammoniumhydrogensulfat wurden bei Raumtemperatur zunächst 476,74 g (2,74 mol) Natriumdithionit und anschließend 250 g (2,68 mol) Anilin gegeben. Anschließend wurde bei 35-40°C tropfenweise mit einer Lösung von 998,07 g 1,2-Dibrom-1,1,2,3,3,3-hexafluorpropan in 300 ml tert.-Butylmethylether versetzt und nach beendeter Zugabe 18 h bei RT nachgerührt. Es wurde falls notwendig mit Natriumcarbonat auf einen pH-Wert von 5 eingestellt und die organische Phase abgetrennt, getrocknet und eingeengt. Der verbleibende Rückstand wird im Vakuum bei 0.3 mbar bis 100°C Innentemperatur andestilliert

Auf diese Weise wurden 644 g (65 % d. Th.) des Produktes erhalten.

### Beispiel 6

### Herstellung von 2-Chlor-4-(1-brom-1,1,2,3,3,3-hexafluor-2-propyl)-anilin

Zu einer Mischung aus 40,0 g (0,12 mol) 4-(1-Brom-1,1,2,3,3,3-hexafluor-2-propyl)-anilin aus Beispiel 6 und 125 ml Acetonitril wurden bei 60°C 18,24 g (0,14 mol) N-Chlorsuccinimid zugegeben und die resultierende Mischung 3 Stunden unter Rückfluss erhitzt. Anschließend wurde das Lösungsmittel größtenteils abdestilliert, der Rückstand mit 100 ml 1N NaOH-Lösung gewaschen, die organische Phase mit Natriumsulfat getrocknet und abschließend unter vermindertem Druck das restliche Lösungsmittel entfernt.

Auf diese Weise wurden 41 g (81 % d. Th.) des Produktes erhalten.

### Beispiel 7

### Herstellung von 2-Brom-4-(1-brom-1,1,2,3,3,3-hexafluor-2-propyl)-anilin

Zu einer Mischung aus 40,0 g (0,12 mol) 4-(1-Brom-1,1,2,3,3,3-hexafluor-2-propyl)-anilin aus Beispiel 6 und 125 ml Acetonitril wurden bei 60°C 22,11 g (0,12 mol) N-Bromsuccinimid zugegeben und die resultierende Mischung 3 Stunden unter Rückfluss erhitzt. Anschließend wurde das Lösungsmittel größtenteils abdestilliert, der Rückstand mit 100 ml 1N NaOH-Lösung gewaschen, die organische Phase mit Natriumsulfat getrocknet und abschließend unter vermindertem Druck das restliche Lösungsmittel entfernt.

Auf diese Weise wurden 47 g (71 % d. Th.) des Produktes erhalten.

### Beispiel 8

### Herstellung von 2-Fluor-4-(1-brom-1,1,2,3,3,3-hexafluor-2-propyl)-anilin

Zu einer Mischung aus 450 ml Wasser, 225 ml tert.-Butylmethylether, 94,50 g (1,12 mol) Natriumhydrogencarbonat und 18,33 g Tetra-n-butylammoniumhydrogensulfat wurden bei Raumtemperatur zunächst 195,86 g (1,12 mol) Natriumdithionit und anschließend 50 g (0,45 mol) 2-Fluoranilin gegeben. Anschließend wurde bei 35-40°C tropfenweise mit einer Lösung von 348,54 g 1,2-Dibrom-1,1,2,3,3,3-hexafluorpropan in 200 ml tert.-Butylmethylether versetzt und nach beendeter Zugabe über Nacht bei RT nachgerührt. Es wurde mit Natriumcarbonat auf einen pH-Wert von 5 eingestellt und die organische Phase abgetrennt, getrocknet und eingeengt.

Auf diese Weise wurden 25 g (14 % d. Th.) des Produktes erhalten.

## Patentansprüche

1. Verfahren zur Herstellung von Verbindungen der Formel (I), 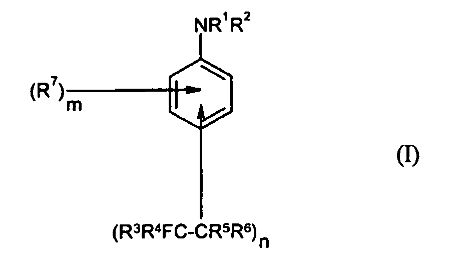 in der
R¹ und R² jeweils unabhängig voneinander für Wasserstoff, C₁-C₁₂-Alkyl, CO(C₁-C₁₂-Alkyl), CO(C₅-C₁₄-Aryl), CO(C₆-C₁₅-Arylalkyl), COO(C₁-C₁₂-Alkyl), COO(C₅-C₁₄-Aryl), COO(C₆-C₁₅-Aryl- alkyl), COO(C₂-C₁₂-Alkenyl) oder C₆-C₁₅-Arylalkyl stehen oder
NR¹R² als Ganzes für einen cyclischen Rest mit insgesamt 4 bis 16 Kohlenstoffatomen oder Isocyanat steht und
R³, R⁴, R⁵ und R⁶ für Fluor oder C₁-C₁₂-Perfluoralkyl stehen und/oder jeweils zwei der Reste R³, R⁴, R⁵ und R⁶ einen cyclischen Per- fluoralkylrest mit insgesamt 4 bis 20 Kohlenstoffatomen bilden und
n für eins oder zwei steht und
R⁷ für C₁-C₁₂-Alkyl, C₅-C₁₄-Aryl, C₆-C₁₅-Arylalkyl, Hydroxy, Chlor, Brom, Fluor, Nitro, Cyano, freies oder geschütztes Formyl, C₁-C₁₂-Halogenalkyl oder Reste der Formeln (IIa) bis (IIf) steht,
A-B-D-E (IIa)
A-E (IIb)
A-SO₂-E (IIc)
A-B-SO₂R⁹ (IId)
A-SO₃ W (IIe)
A-COW (IIf)
in denen unabhängig voneinander
A fehlt oder für einen C₁-C₈-Alkylenrest steht und
B fehlt oder für Sauerstoff, Schwefel oder NR⁸ steht,
wobei
R⁸ Wasserstoff, C₁-C₈-Alkyl, C₆-C₁₅-Arylalkyl oder C₅-C₁₄-Aryl bedeutet und
D für eine Carbonyl-Gruppe steht und
E für R⁹, OR⁹, NHR¹⁰oder N(R¹⁰)₂ steht,
wobei
R⁹ für C₁-C₈-Alkyl, C₆-C₁₅-Arylalkyl, C₁-C₈-Halo- genalkyl oder C₅-C₁₄-Aryl und
R¹⁰ jeweils unabhängig für C₁-C₈-Alkyl, C₆-C₁₅- Arylalkyl oder C₆-C₁₄-Aryl steht oder N(R¹⁰)₂ zusammen für einen cyclischen Aminorest mit 4 bis 12 Kohlenstoffatomen steht und
W für OH, NH₂, oder OM steht, wobei M ein Alkali- metallion, ein halbes Äquivalent eines Erdalkali- metallions, ein Ammoniumion oder ein organisches Ammoniumion bedeuten kann oder zwei Reste R⁷ zusammen einen cyclischen Rest mit insgesamt 5 bis 12 Kohlenstoffatomen bilden können und
m für eine ganze Zahl von 0 bis 5-n steht,
**dadurch gekennzeichnet, dass**
• Verbindungen der Formel (II) 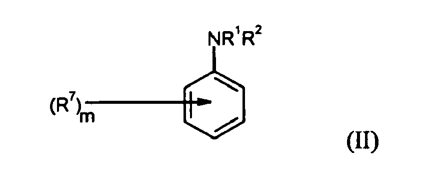 in der
R¹, R², R⁷ und m die vorstehend genannte Bedeutung besitzen
• in einem Schritt a)
mit Verbindungen der Formel (III)
R³R⁴Hal¹C-CHal²R⁵R⁶ (III)
in der
R³, R⁴, R⁵ und R⁶ die vorstehend genannte Bedeutung besitzen und Hal¹ und Hal² jeweils unabhängig voneinander für Chlor, Brom oder Iod stehen,
zu Verbindungen der Formel (IV) umgesetzt werden 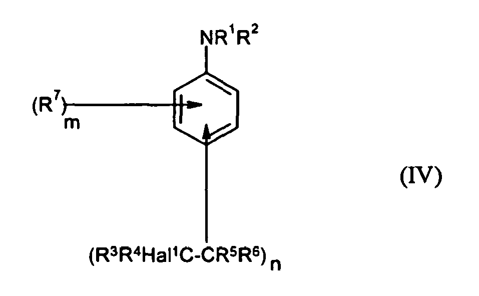 in der
R¹, R², R³, R⁴, R⁵, R⁶ und R⁷ sowie m, n und Hal¹ die vorstehend genannte Bedeutung besitzen und
• in einem Schritt b)
die Verbindungen der Formel (IV) durch Umsetzung mit ionischem Fluorid in Verbindungen der Formel (I) überführt werden.

2. Verfahren nach Anspruch 1, **dadurch gekennzeichnet, dass**
R¹ und R² identisch für Wasserstoff stehen oder
NR¹R² als Ganzes für NHCO(C₁-C₁₂-Alkyl), NHCO(C₅-C₁₄-Aryl) oder NHCO(C₆-C₁₅-Arylalkyl) steht.

3. Verfahren nach mindestens einem der Ansprüche 1 und 2, **dadurch gekennzeichnet, dass**
R³R⁴FC-CR⁵R⁶ als Ganzes für einen sekundären C₃-C₁₂-Perfluor- alkylrest steht.

4. Verfahren nach mindestens einem der Ansprüche 1 bis 3, **dadurch gekennzeichnet, dass**
R⁷ jeweils unabhängig für C₁-C₄-Alkyl, Chlor, Fluor, Nitro, Cyano oder C₁-C₄-Alkoxy steht.

5. Verfahren nach mindestens einem der Ansprüche 1 bis 4, **dadurch gekennzeichnet, dass** n für 1 und m für 0, 1 oder 2 steht.

6. Verfahren nach mindestens einem der Ansprüche 1 bis 5, **dadurch gekennzeichnet, dass** 2-Methyl-4-(heptafluor-2-propyl)-anilin hergestellt wird.

7. Verfahren nach mindestens einem der Ansprüche 1 bis 6, **dadurch gekennzeichnet, dass** die Verbindungen der Formel (III) durch Umsetzung von Verbindungen der Formel (V)
R³-CR⁴=CR⁵-R⁶ (V)
mit Halogenverbindungen der Formel (VI)
Hal¹-Hal² (VI)
umgesetzt werden, wobei R³ , R⁴ , R⁵ und R⁶ sowie Hal¹ und Hal² die unter den Formeln (I) und (III) in den vorstehenden Ansprüchen genannte Bedeutung besitzen.

8. Verfahren nach mindestens einem der Ansprüche 1 bis 7, **dadurch gekennzeichnet, dass** als Verbindungen der Formel (III) 1,2-Dibrom-1,1,2,3,3,3-hexafluorpropan, 2,3-Dibrom-1,1,1,2,3,4,4,4-octafluorbutan, 1,2-Dichlor-3,3,4,4-tetrafluorcyclobutan oder 1,2-Dibrom-1,2,3,3,4,4,5,5-octafluorcyclopentan eingesetzt wird.

9. Verfahren nach mindestens einem der Ansprüche 1 bis 8, **dadurch gekennzeichnet, dass** die Umsetzung im Schritt a) in Gegenwart eines Reduktionsmittels und/oder in Gegenwart Licht mit einer Wellenlänge von 400 nm oder weniger erfolgt.

10. Verfahren nach Anspruch 9, **dadurch gekennzeichnet, dass** als Reduktionsmittel Alkalimetalldithionite eingesetzt werden.

11. Verfahren nach mindestens einem der Ansprüche 1 bis 10, **dadurch gekennzeichnet, dass** die Reaktion in einem mehrphasigen Reaktionsmedium durchgeführt wird, das eine wässrige und zumindest eine organische Phase aufweist.

12. Verfahren nach mindestens einem der Ansprüche 1 bis 11, **dadurch gekennzeichnet, dass** die Reaktion gemäß Schritt a) in Gegenwart von Phasentransferkatalysator durchgeführt wird.

13. Verfahren nach mindestens einem der Ansprüche 1 bis 12, **dadurch gekennzeichnet, dass** die Reaktionstemperatur im Schritt a) -10°C bis zum Siedepunkt des Reaktionsmediums unter Reaktionsdruck beträgt, wobei der Reaktionsdruck 0,5 bis 100 bar beträgt.

14. Verfahren nach mindestens einem der Ansprüche 1 bis 13, **dadurch gekennzeichnet, dass** Schritt a) in Gegenwart einer Base durchgeführt wird.

15. Verfahren nach mindestens einem der Ansprüche 1 bis 14, **dadurch gekennzeichnet, dass** Verbindungen der Formel (IV) die in ortho-Position zur Aminogruppe Chlor oder Brom tragen so hergestellt werden, dass die entsprechenden in ortho-Position unsubstituierten Verbindungen gemäß Schritt a) umgesetzt werden und vor dem Schritt b) halogeniert werden.

16. Verfahren nach mindestens einem der Ansprüche 1 bis 15, **dadurch gekennzeichnet, dass** in Schritt b) als ionische Fluoride quartäre Ammonium- oder Phosphoniumfluoride sowie Alkalimetallfluoride oder Mischungen der genannten Verbindungen oder Mischungen von Phasentransferkatalysatoren und/oder Halex-Katalysatoren mit Alkalimetallfluoriden eingesetzt werden.

17. Verfahren nach mindestens einem der Ansprüche 1 bis 16, **dadurch gekennzeichnet, dass** im Schritt b) Natrium-, Kalium- und Cäsiumfluorid oder Mischungen davon eingesetzt werden.

18. Verfahren nach mindestens einem der Ansprüche 1 bis 17, **dadurch gekennzeichnet, dass** Schritt b) in Gegenwart von organischen Lösungsmittel durchgeführt wird.

19. Verfahren nach Anspruch 18, **dadurch gekennzeichnet, dass** der Wassergehalt des Lösungsmittels maximal 1 Gew.-% beträgt.

20. Verfahren nach mindestens einem der Ansprüche 1 bis 19, **dadurch gekennzeichnet, dass** die Reaktionstemperatur im Schritt b) 60°C bis zum Siedepunkt des eingesetzten Lösungsmittel bei Reaktionsdruck maximal jedoch 300°C beträgt, wobei der Reaktionsdruck 0,5 bis 100 bar beträgt.

## Claims

1. Process for preparing compounds of the formula (I) 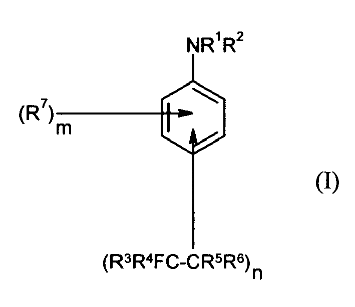 in which
R¹ and R² are each independently hydrogen, C₁-C₁₂-alkyl, CO(C₁-C₁₂- alkyl), CO(C₅-C₁₄-aryl), CO(C₆-C₁₅-arylalkyl), COO(C₁-C₁₂- alkyl), COO(C₅-C₁₄-aryl), COO(C₆-C₁₅-arylalkyl), COO(C₂-C₁₂- alkenyl) or C₆-C₁₅-arylalkyl, or
NR¹R² as a whole is a cyclic radical having a total of 4 to 16 carbon atoms, or is isocyanate, and
R³, R⁴, R⁵ and R⁶ are each fluorine or C₁-C₁₂-perfluorolkyl, and/or in each case two of the R³, R⁴, R⁵ and R⁶ radicals each form a cyclic perfluoroalkyl radical having a total of 4 to 20 carbon atoms, and
n is one or two, and
R⁷ is C₁-C₁₂-alk_{Y}l, C₅-C₁₄-aryl, C₆-C₁₅-arylalkyl, hydroxyl, chlorine, bromine, fluorine, nitro, cyano, free or protected formyl, C₁-C₁₂- haloalkyl, or radicals of the formulae (IIa) to (IIf),
A-B-D-E (IIa)
A-E (IIb)
A-SO₂-E (IIc)
A-B-SO₂R⁹ (IId)
A-SO₃ W (IIe)
A-COW (IIf)
in which, each independently,
A is absent or is a C₁-C₈-alkylene radical and
B is absent or is oxygen, sulphur or NR⁸,
R⁸ is hydrogen, C₁-C₈-alkyl, C₆-C₁₅-arylalkyl or C₅- C₁₄-aryl, and
D is a carbonyl group and
E is R⁹, OR⁹, NHR¹⁰ or N(R¹⁰)₂
R⁹ is C₁-C₈-alkyl, C₆-C₁₅-arylalkyl, C₁-C₈-haloalkyl or C₅-C₁₄-aryl, and
R¹⁰ is in each case independently C₁-C₈-alkyl, C₆-C₁₅- arylalkyl or C₆-C₁₄-aryl, or N(R¹⁰)₂ together is a cyclic amino radical having 4 to 12 carbon atoms and
W is OH, NH₂ or OM where M is an alkali metal ion, half an equivalent of an alkaline earth metal ion, an ammonium ion or an organic ammonium ion, or two R⁷ radicals together may form a cyclic radical having a total of 5 to 12 carbon atoms, and
m is an integer from 0 to 5-n,
**characterized in that**
• compounds of the formula (II) 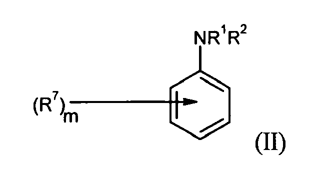 in which
R¹, R², R⁷ and m are each as defined above
• are reacted in a step a)
with compounds of the formula (III)
R³R⁴Hal¹C-CHal²R⁵R⁶ (III)
in which
R³, R⁴, R⁵ and R⁶ are each as defined above and
Hal¹ and Hal² are each independently chlorine, bromine or iodine,
to give compounds of the formula (IV) 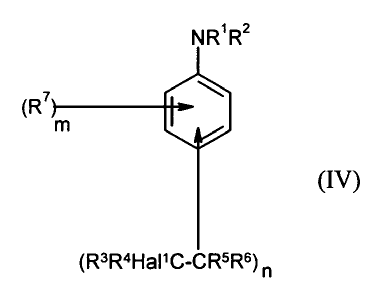 in which
R¹, R², R³, R⁴, R⁵, R⁶ and R⁷, and also m, n and Hal¹, are each as defined above and
• in a step b),
the compounds of the formula (IV) are converted to compounds of the formula (I) by reacting with ionic fluoride.

2. Process according to Claim 1, **characterized in that**
R¹ and R² are identically hydrogen or
NR¹R² as a whole is NHCO(C₁-C₁₂-alkyl), NHCO(C₅-C₁₄-aryl) or NHCO(C₆-C₁₅-arylalkyl).

3. Process according to at least one of Claims 1 and 2, **characterized in that**
R³R⁴FC-CR⁵R⁶ as a whole is a secondary C₃-C₁₂-perfluoroalkyl radical.

4. Process according to at least one of Claims 1 to 3, **characterized in that**
R⁷ is in each case independently C₁-C₄-alkyl, chlorine, fluorine, nitro, cyano or C₁-C₄-alkoxy.

5. Process according to at least one of Claims 1 to 4, **characterized in that** n is 1 and m is 0, 1 or 2.

6. Process according to at least one of Claims 1 to 5, **characterized in that** 2-methyl-4-(heptafluoro-2-propyl)aniline is prepared.

7. Process according to at least one of Claims 1 to 6, **characterized in that** the compounds of the formula (III) are converted by reacting compounds of the formula (V)
R³-CR⁴=CR⁵-R⁶ (V)
with halogen compounds of the formula (VI)
Hal¹-Hal² (VI)
where R³, R⁴, R⁵ and R⁶, and also Hal¹ and Hal², are each as defined under formulae (I) and (III) in the above claims.

8. Process according to at least one of Claims 1 to 7, **characterized in that** the compounds of the formula (III) used are 1,2-dibromo-1,1,2,3,3,3-hexafluoropropane, 2,3-dibromo-1,1,1,2,3,4,4,4-octafluorobutane, 1,2-dichloro-3,3,4,4-tetrafluorocyclobutane or 1,2-dibromo-1,2,3,3,4,4,5,5-octafluorocyclopentane.

9. Process according to at least one of Claims 1 to 8, **characterized in that** the reaction in step a) is effected in the presence of a reducing agent and/or in the presence of light having a wavelength of 400 nm or less.

10. Process according to Claim 9, **characterized in that** the reducing agents used are alkali metal dithionites.

11. Process according to at least one of Claims 1 to 10, **characterized in that** the reaction is carried out in a multiphase reaction medium which has one aqueous and at least one organic phase.

12. Process according to at least one of Claims 1 to 11, **characterized in that** the reaction in step a) is carried out in the presence of a phase transfer catalyst.

13. Process according to at least one of Claims 1 to 12, **characterized in that** the reaction temperature in step a) is -10°C up to the boiling point of the reaction medium under reaction pressure, the reaction pressure being 0.5 to 100 bar.

14. Process according to at least one of Claims 1 to 13, **characterized in that** step a) is carried out in the presence of a base.

15. Process according to at least one of Claims 1 to 14, **characterized in that** compounds of the formula (IV) which bear chlorine or bromine in the ortho-position to the amino group are prepared in such a way that the corresponding compounds unsubstituted in the ortho-position are converted in step a) and halogenated before step b).

16. Process according to at least one of Claims 1 to 15, **characterized in that** the ionic fluorides used in step b) are quaternary ammonium fluorides or phosphonium fluorides, or else alkali metal fluorides or mixtures of the compounds mentioned or mixtures of phase transfer catalysts and/or halex catalysts with alkali metal fluorides.

17. Process according to at least one of Claims 1 to 16, **characterized in that** sodium fluoride, potassium fluoride and caesium fluoride or mixtures thereof are used in step b).

18. Process according to at least one of Claims 1 to 17, **characterized in that** step b) is carried out in the presence of organic solvent.

19. Process according to Claim 18, **characterized in that** the maximum water content of the solvent is 1% by weight.

20. Process according to at least one of Claims 1 to 19, **characterized in that** the reaction temperature in step b) is 60°C up to the boiling temperature of the solvent used at reaction pressure, but a maximum of 300°C, the reaction pressure being 0.5 to 100 bar.

## Revendications

1. Procédé de préparation de composés de la formule (I), 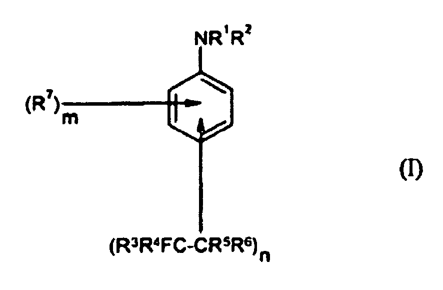 dans laquelle
R¹ et R² sont chaque fois indépendamment l'un de l'autre un hydrogène, alkyle(C₁-C₁₂), CO- alkyle (C₁-C₁₂) , CO-aryle (C₅-C₁₄) , CO- arylalkyle(C₆-C₁₅) , COO-alkyle(C₁-C₁₂) , COO- aryle (C₅-C₁₄), COO-arylalkyle (C₆-C₁₅) , COO- alcényle (C₂-C₁₂) ou arylalkyle(C₆-C₁₅), ou
NR¹R² est globalement un radical cyclique avec au total 4 à 16 atomes de carbone ou un isocyanate, et
R³, R⁴, R⁵ et R⁶ sont un fluor ou perfluoroalkyle(C₁-C₁₂) et/ou chaque fois deux des radicaux R³, R⁴, R⁵ et R⁶ forment un radical perfluoroalkyle cyclique avec au total 4 à 20 atomes de carbone et
n est 1 ou 2 et
R⁷ est un alkyle (C₁-C₁₂) , aryle (C₅-C₁₄) , arylalkyle(C₆-C₁₅), hydroxy, chlore, brome, fluor, nitro, cyano, formyle libre ou protégé, halogénalkyle(C₁-C₁₂) ou des radicaux des formules (IIa) à (IIf),
A-B-D-E (IIa)
A-E (IIb)
A-SO₂-E (IIc)
A-B-SO2R9 (IId)
A-SO3W (IIe)
A-COW (IIf)
dans lesquelles indépendamment l'un de l'autre
A manque ou est un radical alkylène(C₁-C₈) et
B manque ou est un oxygène, soufre ou NR⁸,
dans laquelle
R⁸ est un hydrogène, alkyle(C₁-C₈), arylalkyle(C₆- C₁₅) ou aryle (C₅-C₁₄) et
D est un groupe carbonyle et
E est R⁹, OR⁹, NHR¹⁰ ou N(R¹⁰)₂,
dans laquelle
R⁹ est un alkyle (C₁-C₈) , arylalkyle (C₆-C₁₅) , halogénalkyle (C₁-C₈) ou aryle(C₅-C₁₄) et
R¹⁰ est chaque fois indépendamment un alkyle (C₁-C₈) , arylalkyle (C₆-C₁₅) ou aryle (C₆-C₁₄) ou N (R¹⁰) ₂ est ensemble un radical amino cyclique avec 4 à 12 atomes de carbone et
W est un OH, NH₂, ou OM, dans laquelle M peut être un ion de métal alcalin, un demi- équivalent d'un ion de métal alcalino-terreux, un ion ammonium ou un ion ammonium organique ou deux radicaux R⁷ peuvent former ensemble un radical cyclique avec au total 5 à 12 atomes de carbone et
m est un nombre entier de 0 à 5-n,
**caractérisé en ce que**
• on convertit des composés de la formule (II) 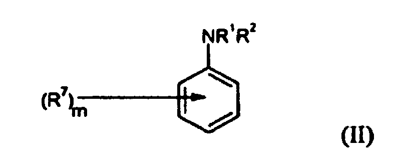 dans laquelle
R¹, R², R⁷ et m ont la signification mentionnée plus haut
• dans une étape a)
avec des composés de la formule (III)
R³R⁴Hal¹C-CHal²R⁵R⁶ (III) dans laquelle
R³, R⁴, R⁵ et R⁶ ont la signification mentionnée plus haut et
Hal¹ et Hal² sont chaque fois indépendamment l'un de l'autre un chlore, brome ou iode,
en composés de la formule (IV) 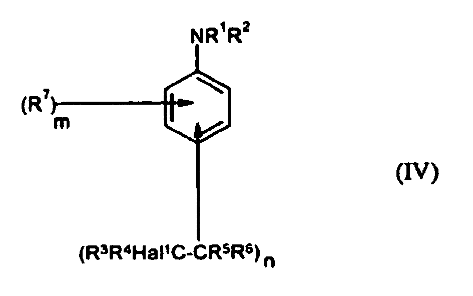 dans laquelle
R¹, R², R³, R⁴, R⁵, R⁶ et R⁷ ainsi que m, n et Hall ont la signification mentionnée plus haut et
• dans une étape b)
on transforme les composés de la formule (IV) en composés de la formule (I) par conversion avec un fluorure ionique.

2. Procédé selon la revendication 1, **caractérisé en ce que**
R¹ et R² sont identiques et sont un hydrogène ou
NR¹R² est globalement un NHCO-alkyle (C₁-C₁₂) , NHCO- aryle (C₅-C₁₄) ou NHCO-arylalkyle (C₆-C₁₅).

3. Procédé selon au moins une des revendications 1 et 2, **caractérisé en ce que**
R³R⁴FC-CR⁵R⁶ est globalement un radical secondaire perfluoroalkyle (C₃-C₁₂) .

4. Procédé selon au moins une des revendications 1 à 3, **caractérisé en ce que**
R⁷ est chaque fois indépendamment un alkyle(C₁- C₄), chlore, fluor, nitro, cyano ou alcoxy(C₁- C₄).

5. Procédé selon au moins une des revendications 1 à 4, **caractérisé en ce que** n est égal à 1 et m est égal à 0, 1 ou 2.

6. Procédé selon au moins une des revendications 1 à 5, **caractérisé en ce que** l'on prépare du 2-méthyl-4-(heptafluor-2-propyl)-aniline.

7. Procédé selon au moins une des revendications 1 à 6, **caractérisé en ce que** l'on convertit les composés de la formule (III) par conversion de composés de la formule (V)
R³-CR⁴=CR⁵-R⁶ (V)
avec des composés halogénés de la formule (VI)
Hal¹-Hal² (VI),
dans lesquelles R³, R⁴, R⁵ et R⁶ ainsi que Hall et Hal² ont la signification indiquée dans les formules (I) et (III) dans les revendications précédentes.

8. Procédé selon au moins une des revendications 1 à 7, **caractérisé en ce que** l'on utilise comme composés de la formule (III) le 1,2-dibrome-1,1,2,3,3,3-hexafluoropropane, le 2,3-dibrome-1,1,1,2,3,4,4,4-octafluorobutane, le 1,2-dichloro-3,3,4,4-tétrafluorocyclobutane ou le 1,2-dibrome-1,2,3,3,4,4,5,5-octafluorocyclopentane.

9. Procédé selon au moins une des revendications 1 à 8, **caractérisé en ce que** l'on effectue la conversion de l'étape a) en présence d'un agent réducteur et/ou en présence de lumière avec une longueur d'onde de 400 nm ou moins.

10. Procédé selon la revendication 9, **caractérisé en ce que** l'on utilise comme agent réducteur des dithionites de métal alcalin.

11. Procédé selon au moins une des revendications 1 à 10, **caractérisé en ce que** l'on effectue la réaction dans un milieu de réaction polyphasique, qui présente une phase aqueuse et au moins une phase organique.

12. Procédé selon au moins une des revendications 1 à 11, **caractérisé en ce que** l'on effectue la réaction selon l'étape a) en présence de catalyseur de transfert de phase.

13. Procédé selon au moins une des revendications 1 à 12, **caractérisé en ce que** la température de réaction à l'étape a) vaut de -10°C au point d'ébullition du milieu de réaction à la pression de réaction, dans lequel la pression de réaction vaut de 0,5 à 100 bar.

14. Procédé selon au moins une des revendications 1 à 13, **caractérisé en ce que** l'on exécute l'étape a) en présence d'une base.

15. Procédé selon au moins une des revendications 1 à 14, **caractérisé en ce que** l'on prépare des composés de la formule (IV), qui portent un chlore ou un brome en position ortho par rapport au groupe amino, de telle manière que les composés correspondants non substitués en position ortho soient convertis selon l'étape a) et soient halogénés avant l'étape b).

16. Procédé selon au moins une des revendications 1 à 15, **caractérisé en ce que** l'on utilise comme fluorures ioniques à l'étape b) des fluorures quaternaires d'ammonium ou de phosphonium ainsi que des fluorures de métaux alcalins ou des mélanges des composés précités ou des mélanges de catalyseurs de transfert de phase et/ou de catalyseurs Halex avec des fluorures de métaux alcalins.

17. Procédé selon au moins une des revendications 1 à 16, **caractérisé en ce que** l'on utilise à l'étape b) du fluorure de sodium, de potassium et de césium ou des mélanges de ceux-ci.

18. Procédé selon au moins une des revendications 1 à 17, **caractérisé en ce que** l'on exécute l'étape b) en présence de solvant organique.

19. Procédé selon la revendication 18, **caractérisé en ce que** la teneur en eau du solvant vaut au maximum 1 % en poids.

20. Procédé selon au moins une des revendications 1 à 19, **caractérisé en ce que** la température de réaction à l'étape b) vaut de 60°C au point d'ébullition du solvant utilisé à la pression de réaction, mais vaut cependant au maximum 300°C, dans lequel la pression de réaction vaut 0,5 à 100 bar.
